Europäisches Patentamt

European Patent Office

Office européen des brevets

(19)

(11) Numéro de publication: **0 309 310 B1**

(12) **FASCICULE DE BREVET EUROPEEN**

(45) Date de publication de fascicule du brevet: **16.12.92**

(51) Int. Cl.5: **C12P 13/04**, C12N 9/86, //C12N9/90

(21) Numéro de dépôt: **88402259.1**

(22) Date de dépôt: **08.09.88**

(54) **Nouveau système enzymatique, son procédé de préparation et son application notamment dans la préparation de la D-parahydroxyphénylglycine.**

(30) Priorité: **21.09.87 FR 8713030**

(43) Date de publication de la demande:
**29.03.89 Bulletin 89/13**

(45) Mention de la délivrance du brevet:
**16.12.92 Bulletin 92/51**

(84) Etats contractants désignés:
**AT BE CH DE ES FR GB GR IT LI LU NL SE**

(56) Documents cités:
**FR-A- 2 431 536**
**GB-A- 2 042 531**

**BIOTECHNOLOGY AND BIOENGINEERING, vol. 13, no. 10, octobre 1981, pages 2173-2183, John Wiley & Sons, Inc., New York, US; R. OLIVIERI et al.: "Microbial transformation of racemic hydantoins to D-amino acids"**

**CHEMICAL ABSTRACTS, vol. 90, no. 17, avril 1979, page 379, résumé no. 136254b, Columbus, Ohio, US; && JP-A-78 136 583 (KANEGAFUCHI CHEMICAL INDUSTRY CO.**

LTD) 29-11-1978

**AGRICULTURAL AND BIOLOGICAL CHEMISTRY, vol. 51, no. 2, février 1987, pages 355-362, Tokyo, JP; K. YOKOZEKI et al.: "Screening of microorganisms producing D-p-hydroxyphenylglycine from DL-5-(p-hydroxyphenyl)hydantoin"**

(73) Titulaire: **SOCIETE FRANCAISE HOECHST Société anonyme dite:**
**3, avenue du Général de Gaulle**
**F-92800 Puteaux(FR)**

(72) Inventeur: **Ohleyer, Eric**
**Hery sur Alby**
**F-74430 Alby sur Cheran(FR)**

(74) Mandataire: **Rinuy, Santarelli**
**14, avenue de la Grande Armée**
**F-75017 Paris(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

La présente invention concerne un nouveau système enzymatique, son procédé de préparation et son application à la préparation par voie enzymatique de la D-parahydroxyphénylglycine.

Aujourd'hui, on utilise de plus en plus des procédés enzymatiques pour accéder à des substances présentant une configuration déterminée sans qu'il soit nécessaire d'effectuer des séparations fastidieuses d'énantiomères ou de diastéréoisomères. Tel est le cas de la D-parahydroxyphénylglycine, désignée ci-après PHPG, qui est un amino-acide couramment utilisé dans l'élaboration d'agents antibactériens.

Pour l'obtenir, on a recours, très souvent, à la D,L-parahydroxyphényl-5 hydantoïne, désignée ci-après PHPH.

Le système enzymatique objet de la présente invention permet de transformer par voie enzymatique de la PHPH en PHPG.

On sait que la parahydroxyphényl-5 hydantoïne racémique peut être hydrolysée par voie enzymatique, en acide D-alpha uréidoparahydroxyphénylacétique, hydrolysable ensuite, par voie chimique, en PHPG (cf. notamment les brevets français 2.310.986, 2.317.357, 2.383.961, la demande de brevet allemand N° 27 57 980 et les demandes de brevet japonais N78-44690, 80-104890, 81-23892 et 86-177991).

On sait par ailleurs que certains systèmes enzymatiques n'hydrolysent que l'isomère D de la parahydroxyphényl-5 hydantoïne en PHPG (cf. brevet français 2.393.848 et demande de brevet japonais 86-177991).

Enfin, il est connu que certains autres systèmes enzymatiques issus de micro-organismes du type Hansenula, Pseudomonas Arthrobacter, Alcaligenes, Achromobacter Moraxella, Flavobacterium, Agrobacterium sont susceptibles de transformer la PHPH en PHPG (cf. notamment brevet français 2.438.087, 2.456.728, demande de brevet européen 0.199.943 et demandes de brevet japonais 79-89088, 80-114291, 80-114292 et 86-181391).

Toutefois, ces systèmes enzymatiques permettant de transformer directement la PHPH en PHPG fournissent généralement de la PHPG soit avec un faible rendement, soit avec une faible productivité. De plus, ces systèmes enzymatiques travaillent en fait à un pH supérieur à 7, ce qui entraîne des colorations parasites altérant la PHPG obtenue et nécessitant des purifications ultérieures contraignantes qui affectent grandement l'emploi de la PHPG obtenue par ces systèmes dans la synthèse d'antibiotiques semi-synthétiques dont c'est l'une de ses applications essentielles.

Or la Demanderesse a découvert avec étonnement un nouveau système enzymatique remédiant à ces inconvénients et permettant d'obtenir directement avec des rendements et une productivité satisfaisants une PHPG pure et incolore.

Le système enzymatique selon la présente invention est obtenu par culture d'un micro-organisme, identifié plus complètement ci-après, dans un milieu convenable.

Le micro-organisme dont la culture dans des conditions appropriées fournit le système enzymatique selon l'invention, doit être considéré comme une espèce qui appartient au genre Agrobacterium.

Il a été isolé à partir d'un échantillon de boue prélevée à Stains (France) selon les méthodes habituelles d'isolement des micro-organismes. Un échantillon de cette souche a été déposé à la Collection Nationale de Cultures de Micro-organismes, Institut Pasteur, 75724 Paris Cédex 15, où il a été enregistré sous le numéro I-671, le 29 juin 1987.

Ce micro-organisme, dénommé Agrobacterium sp EO III 50, désigné par la suite EO III 50 P, est un Agrobacterium très proche de l'espèce tumefaciens. A la connaissance de la Demanderesse, cette souche n'est pas décrite dans la littérature.

Les caractères culturaux et morphologiques de ce micro-organisme sont les suivants :

### Eléments de base

- bacilles de diamètre moyen, de forme homogène, sans arrangement notable,
- mobilité + + (1-3 flagelles d'implantation peritriche de 2 à 4 longueurs d'onde - imprégnation aux sels d'aluminium),
- coloration de Gram -,
- lyse par le KOH 3 % + + +,
- aminopeptidase (CZERNY) + + +,
- culture apparente en gélose profonde : aérobie obligatoire,
- culture luxuriante sur géloses ordinaires : colonies opalescentes, étendues, d'un diamètre de 3-5 mm, aux contours et à surfaces lisses, légèrement confluentes entre elles (gélose Columbia, 30°, atmosphère ambiante, 4 jours d'incubation),

- culture en bouillon : NaCl 0 % + + +,
- culture en bouillon : NaCl 2 % + + +,
- culture en bouillon : NaCl 4 % + + +,
- culture en bouillon : NaCl 6 % -,
- culture en bouillon : NaCl 8 % -,
- culture en bouillon : NaCl 10 % -
 (milieu de base bouillon PYG),
- culture à 10° + +,
- culture à 30° + + +,
- culture à 41° -,
 (milieu de base gélose Columbia),
- culture prototrophe,
- pigment -,
- catalase +,
- réaction d'oxydase + + +,
- production acide en aérobiose à partir du glucose +,
- production acide en anaérobiose à partir du glucose -,
- nitrate réductase + + +,
- nitrite réductase + + +,
- production de gaz à partir des nitrates -,
- production de gaz à partir des nitrites -,
- culture en anaérobiose en présence de nitrates +,
- thiosulfate réductase -,
- tétrathionate réductase -,
- uréase + + +,
- phénylalanine désaminase -,
- "arginine dihydrolase" -,
- "lysine décarboxylase" -,
- c-glutamyl-transférase + + +,
- accumulation de polyhydroxybutyrate + +,
- production d'oxo-3 lactose -,
- hydrolyse de l'esculine + + +,
- hydrolyse de l'oNPb-galactoside + + +,
- hydrolyse du pNPb-xyloside + + +,
- hydrolyse de l'amidon insoluble -,
- hydrolyse du Tween 80 -,
- lipase sur gélose au jaune d'oeuf -,
- lécithinase -,
- DNAse extracellulaire -,
- gélatinase (FRAZIER) -,
- caséinase -,
- culture en milieu minéral M63, en présence de :
 . glycérol + + +,
 . mannitol + + +,
 . inositol + + +,
 . D-xylose + + +,
 . L-arabinose + + +,
 . D-glucose + + +,
 . saccharose + + +,
 . raffinose + + +,
 . mélibiose + + +,
 . salicine -,
 . lactose + + +,
 . acétamide -,
 . ac. acétique + + +,
 . ac. hydroxy-3-butyrique + + +,
 . ac. adipique -,
 . ac. citrique + + +,

. ac. DL-amino-2-butyrique -,

. ac. L-aspartique +,

. L-arginine + + +,

. L-tryptophane -,

. ac. hydroxy-3-benzoïque +,

. ac. hydroxy-4-benzoïque + + +,

. ac. amino-4-benzoïque -,

. lysine -,

- réaction à 30°, atmosphère ambiante.

Explication des symboles

- :      réaction ou culture négative,

+ :      positive,

+ + + :  particulièrement intense,

Ac :     acide.

Eléments chimiotaxonomiques

**Composition en lipides polaires**

Analyse comparative par chromatographie sur couche mince de silice.
Procédé d'extraction : chloroforme/méthanol 1:2
Chromatographie monodimensionnelle :
- solvant de migration : chloroforme/méthanol/eau/acide acétique : 65:25:4:0.8
- révélation des phospholipides :
  . molybdate d'ammonium,
- des lipides aminés :
  . ninhydrine,
- des glycolipides :
  . alpha-naphtol,
- des lipides totaux :
  . vanilline/$H_2SO_4$.

Souche de référence :

- Agrobacterium tumefaciens CIP 67.1 (mfl 145)
- absence de lipides glycosylés décelables.

Electrophorèse comparative d'un extrait protéique

**Conditions de l'analyse électrophorétique**

Préparation de l'échantillon

Extraction des protéines solubles par chauffage 10 minutes à 100°C en présence de sodium dodécylsulfate à 2 %, à partir de 100 mg environ de pâte bactérienne.

Electrophorèse

- matériel             : cuve verticale BIORAD PROTEAN II
                         : alimentation courant continu modèle BIORAD 500/200
- gel                  : gel discontinu de polyacrylamide de 1,5 mm d'épaisseur et 16 cm de longueur,
                         1er gel : 4 % pH 6,8
                         2me gel : 7 % pH 8,8
- courant              : 35 mA à débit constant pendant cinq heures
- température de la cuve : 10°C

4

EP 0 309 310 B1

- coloration du gel : bleu de Coomassie R-250.

Souches de références :

- Agrobacterium tumefaciens CIP 67.1
- Agrobacterium SP groupe VD CIP 82.11

La souche EO III-50-P est très proche d'Agrobacterium tumefaciens (souche type : CIP 67-1) ; mais elle s'en différencie essentiellement par les profils protéiques comparatifs.

Lorsque l'on cultive la souche EO-III-50-P dans des milieux de culture classiques, le système enzymatique objet de la présente invention, se forme essentiellement dans les cellules du micro-organisme.

Ces milieux de culture doivent contenir essentiellement des sources de carbone et d'azote assimilables ainsi que d'autres éléments nutritifs nécessaires au développement de la souche EO-III-50-P, tels que des sels minéraux. La production du système enzymatique n'est pas sensiblement améliorée si on ajoute au milieu de culture, comme source d'azote, une hydantoïne substituée en position 5 telle que la PHPH.

Par contre, il est avantageux d'introduire, dans le milieu de culture, un inducteur d'activité hydantoïnase non métabolisable. De tels inducteurs sont notamment : l'uracile, le thio-2-uracile, l'aza-6-uracile, la thymine, l'imidazolidinethione, la sec-butyl-thiohydantoïne, l'orotate de sodium. Ces inducteurs sont avantageusement utilisés à une dose comprise entre 1 mM et 10 mM.

Comme source de carbone assimilable, on peut utiliser des hydrates de carbone tels que le glucose, le saccharose, le glycérol, le lactose ou le maltose sous forme pure ou apportés sous forme de résidus complexes tels que les mélasses, le lactosérum ; on peut également utiliser des alcools ou des acides organiques.

Les sources convenables d'azote assimilables sont extrêmement variées. Elles peuvent être des substances chimiques très simples comme l'urée ou des sels minéraux ou organiques d'ammonium. Elles peuvent être aussi apportées par des substances complexes contenant principalement l'azote sous forme protidique, telles que peptones, extraits de levure ou extraits de viande.

Certains éléments minéraux peuvent apporter l'équilibre ionique nécessaire au développement de la souche EO III-50-P, comme les chlorures ou sulfates de métaux alcalins ou alcalino-terreux ou les sels de zinc, de cobalt, de fer, de cuivre, de manganèse, de magnésium. Le pH initial du milieu de fermentation de la culture doit être compris entre 4 et 9, de préférence entre 6 et 8, et avantageusement entre 7 et 7,1.

La température optimale pour la fermentation est de 35°C mais une production satisfaisante est encore obtenue pour des températures comprises entre 20 et 45°C.

L'aération de la fermentation peut varier entre des valeurs assez larges. On a cependant trouvé que des aérations de 0,75 à 2,5 litres d'air par litre de bouillon et par minute conviennent particulièrement bien. Le rendement maximal est obtenu après 8 à 48 heures de culture, ce temps dépendant essentiellement du milieu utilisé.

La présente invention a également pour objet l'application du système enzymatique précédemment décrit à la transformation par voie enzymatique de la PHPH en PHPG.

Selon cette application, la PHPG est préparée en mettant en contact la PHPH avec le système enzymatique de l'invention qui est capable de transformer directement les deux épimères de cette hydantoïne en PHPG sans qu'il soit nécessaire d'opérer à un pH alcalin afin d'effectuer une racémisation chimique.

Le système enzymatique étant produit de manière intracellulaire, son utilisation pour la transformation de la PHPH en PHPG est réalisée selon les méthodes connues de l'homme du métier telles que l'emploi soit d'une suspension de cellules en survie, soit d'un extrait cellulaire obtenu après désintégration des cellules par sonication ou par utilisation d'un "French press", soit par des cellules lyophilisées ou déshydratées à l'aide d'un solvant organique tel que l'acétone, le toluène, soit par des cellules immobilisées selon les techniques classiques.

Le système enzymatique peut être également utilisé sous une forme purifiée ou partiellement purifiée selon les techniques habituelles du génie enzymatique.

La transformation de la PHPH en PHPG par le système enzymatique décrit précédemment est réalisée sous des conditions anaérobies, à un pH compris entre 6 et 8, de préférence à un pH voisin de 6,8 (qui peut être régulé à cette valeur au cours de la transformation par addition d'un agent neutralisant adéquat), à une température comprise entre 20 et 65°C, de préférence à une température voisine de 50°C, généralement sous une agitation modérée et en suspension dans un milieu aqueux constitué habituellement soit par une solution aqueuse isotonique de chlorure de sodium 0,1M, soit par un tampon phosphate pH = 6,7, 0,1M.

La concentration en hydantoïne dans le milieu réactionnel est généralement voisine de 20 g/litre mais

5

elle peut être inférieure ou supérieure.

Généralement, la durée de réaction est comprise entre 2 heures et 4 jours, mais en fait, elle est poursuivie jusqu'à ce qu'il n'y ait plus de PHPH de départ.

Le milieu réactionnel peut également contenir des additifs, classiques pour ce type de réaction, tels que des agents tensioactifs, anti-oxydants, anti-mousses, qui peuvent favoriser la formation de la PHPG et permettre ainsi une amélioration du rendement.

Non seulement le système enzymatique décrit précédemment hydrolyse la D-parahydroxyphényl-5 hydantoïne en D-parahydroxyphénylglycine, mais également racémise in situ la L-parahydroxyhényl-5 hydantoïne au fur et à mesure de l'hydrolyse de son épimère. De ce fait, l'application du système enzymatique de la présente invention permet une transformation complète de la D,L-parahydroxyphényl-5 hydantoïne en PHPG optiquement pure.

Selon une variante, la transformation de la PHPH en PHPG par le système enzymatique produit par la souche EO III-50-P est effectuée en présence de co-solvants miscibles à l'eau tels que le diméthylsulfoxyde, le méthoxy-2-éthanol, le diméthylformamide. Ces co-solvants sont utilisés à une concentration inférieure à 5 % en poids par rapport au poids total du milieu réactionnel.

La PHPG obtenue selon l'application de la présente invention peut être isolée du milieu réactionnel selon les méthodes habituelles de séparation des amino-acides, généralement après clarification du milieu réactionnel par centrifugation et filtration sur une résine échangeuse d'ions. Généralement, on effectue une précipitation de l'amino-acide à son point isolélectrique.

Les exemples suivants, donnés à titre non limitatif, montrent comment l'invention peut être mise en pratique.

Exemple 1

**1-1 Préparation du système enzymatique**

On prépare un milieu de culture aqueux, désigné par la suite A, ayant la composition suivante :

| - glycérol | 10 g/l |
|---|---|
| - extrait de levure | 5 g/l |
| - extrait de viande | 5 g/l |
| - peptone pancréatique digérée | 5 g/l |
| - phosphate monopotassique | 1 g/l |
| - sulfate de manganèse | 0,02 g/l |
| - sulfate de magnésium | 0,1 g/l |
| - sulfate ferreux | 0,02 g/l |
| - sulfate cuivrique | 0,02 g/l |
| - sulfate de calcium | 0,02 g/l |
| - chlorure de sodium | 0,5 g/l |
| - uracile | 0,5 g/l |

Par ailleurs, on prépare une préculture, B, en ensemençant 0,5 litre du milieu A, préalablement stérilisé pendant 20 minutes à 121°C, avec une anse de la souche EO III-50-P (conservée sur le même milieu en présence de 1,4 % d'agar-agar) puis en laissant se développer cette souche pendant 14 heures à 35°C sous une agitation de 200 tours par minute. On obtient ainsi un milieu présentant une densité optique à 610 nm, de 2,4.

Cette préculture B est ensuite introduite dans un fermenteur contenant cinq litres du milieu A stérilisés in situ pendant 20 minutes à 121°C, puis elle est développée, pendant 9 heures, à 35°C, à un pH régulé entre 7,0 et 7,1, sous une agitation de 450 tours par minute et un taux d'oxygène dissous maintenu à une valeur au moins égale à 30 % de la saturation en air. On obtient ainsi un milieu présentant une densité optique, à 610 nm, d'environ 12,5.

La culture terminée, les cellules sont récupérées puis centrifugées pendant 20 minutes, à 4°C, sous 9000 tours par minute, ensuite lavées avec un tampon phosphate pH = 7,00, 0,1M et enfin centrifugées de nouveau. La boue bactérienne ainsi obtenue, désignée C, est mise en suspension dans 1,5 litre d'une solution aqueuse de chlorure de sodium 0,1M.

**1-2 Préparation de la D-parahydroxyphénylglycine**

Dans un réacteur équipé d'une agitation et thermostaté à 45°C, on place, sous atmosphère d'azote, la suspension de boue bactérienne précédente, puis on introduit 15 g de parahydroxyphényl-5 hydantoïne racémique. Après 12 heures d'incubation sous agitation modérée, en atmosphère d'azote, à 45°C et à pH régulé à 6,75 ± 0,1 par addition d'acide sulfurique 0,1N, on constate la disparition de l'hydantoïne de départ et la présence à une concentration de 8,25 g/l de D-parahydroxyphénylglycine. Le milieu réactionnel est ensuite centrifugé puis on isole la D-parahydroxyphénylglycine du filtrat par précipitation à son point isolélectrique. On obtient ainsi de la D-parahydroxyphénylglycine cristallisée pure présentant une excellente pureté optique, $[\alpha]_D^{25}$ = -161,2°, 1 %, HCl N, (littérature $[\alpha]_D^{25}$ = 157,8° 1 % HCl N).

Exemple 2

Dans un réacteur équipé d'une agitation et thermostaté à 45°C, on place sous atmosphère d'azote :
- la boue bactérienne C obtenue selon l'exemple 1,
- 1,5 litre de tampon phosphate pH = 6-7, 0,1M,
- 30 g de parahydroxyphényl-5 hydantoïne racémique.

Après 24 heures d'incubation, sous agitation modérée, en atmosphère d'azote, à 45°C, et à pH régulé à 6,75 ± 0,1 par addition d'acide sulfurique 0,1N, on constate la disparition de l'hydantoïne de départ et la présence à une concentration de 16,9 g/litre de D-parahydroxyphénylglycine. Après isolement, on constate par mesure de son pouvoir rotatoire que la D-parahydroxyphénylglycine obtenue est optiquement pure.

Exemple 3

**3-1- Préparation du système enzymatique**

On prépare un milieu de culture aqueux ayant la composition suivante :

| | |
|---|---|
| - glycérol | 10 g/l |
| - extrait de levure | 5 g/l |
| - peptone pancréatique digérée | 5 g/l |
| - phosphate monopotassique | 1 g/l |
| - sulfate de magnésium | 0,5 g/l |
| - sulfate ferreux | 0,02 g/l |
| - sulfate de manganèse | 0,02 g/l |
| - thio-2-uracile | 0,13 g/l |

Dans un fermenteur de 7 litres, on stérilise 20 minutes à 121°C, 5 litres du milieu de culture précédent puis on l'ensemence comme dans l'exemple 1 avec une culture préparée dans ce milieu de culture selon la méthode donnée à l'exemple 1. La culture est ensuite développée sous des conditions opératoires identiques à celles utilisées à l'exemple 1.

Après 9 heures de fermentation, on obtient un milieu réactionnel présentant une densité optique, à 610 nm, d'environ 14,0. Les cellules sont ensuite isolées comme dans l'exemple 1 et la boue bactérienne obtenue est mise en suspension dans 2 litres d'une solution aqueuse de chlorure de sodium 0,1M contenant pondéralement 3 % de méthoxy-2-éthanol.

**3-2 Préparation de la D-parahydroxyphénylglycine**

Dans un réacteur équipé d'une agitation et thermostaté à 45°C, on place, sous atmosphère d'azote, la suspension de boue bactérienne précédente, puis on introduit 5 g de parahydroxyphényl-5 hydantoïne racémique.

Après 390 minutes d'incubation sous agitation modérée, en atmosphère d'azote, à 45°C, et à pH régulé à 6,75 ± 0,1 par addition d'acide sulfurique 0,1N, on constate la disparition complète de l'hydantoïne de départ et la présence à une concentration de 4,15 g/l de D-parahydroxyphénylglycine dont la pureté optique est confirmée par une chromatographie en couche mince sur plaque chirale.

Exemple 4

On prépare un milieu de culture aqueux ayant la composition suivante :

| - eau de détrempage de maïs | 15 g/l |
|---|---|
| - saccharose | 10 g/l |
| - chlorure de sodium | 5 g/l |
| - phosphate monopotassique | 1 g/l |

150 ml de ce milieu de culture, ajustés à pH = 7,1 et stérilisés pendant 20 minutes à 121°C, sont ense- mencés avec une anse de la souche EO III-50-P prélevée sur une culture sur milieu solide. Après 24 heures d'incubation, à 35°C, en milieu agité, les cellules sont isolées par centrifugation puis mises en suspension dans 50 ml de tampon phosphate, pH = 6,70, 0,1M. Dans cette suspension agitée, sous atmosphère d'azote, on introduit 250 mg de parahydroxyphényl-5 hydantoïne racémique, puis on laisse se développer la culture à 50°C, en atmosphère d'azote. Après 20 heures de réaction, une analyse d'une prise d'essai montre la disparition complète de l'hydantoïne de départ et la présence à une concentration de 4,15 g/l de D-parahydroxyphénylglycine dont la pureté optique est confirmée par chromatographie sur couche mince sur plaque chirale.

Exemple 5

On prépare un milieu aqueux de culture ayant la composition suivante :

| - eaux de détrempage de maïs | 15 g/l |
|---|---|
| - lactose | 10 g/l |
| - chlorure de sodium | 5 g/l |
| - phosphate monopotassique | 1 g/l |

150 ml de ce milieu, ajustés à pH = 7,1 et stérilisés pendant 20 minutes à 21°C, sont ensemencés avec une anse de la souche EO III-50-P prélevée sur une culture sur milieu solide. Après 24 heures d'incubation à 35°C, en milieu agité, les cellules sont isolées par centrifugation puis reprises comme dans l'exemple 4 par 50 ml de tampon phosphate pH = 6,70, 0,1M. Cette suspension est ensuite désintégrée dans un "French press".

Dans la bouillie cellulaire ainsi obtenue, on introduit ensuite 250 mg de parahydroxyphényl-5 hydantoïne racémique puis on incube cette suspension à 50°C, sous atmosphère d'azote. Après 15 heures d'incubation, l'analyse d'une prise d'essai indique la présence de D-parahydroxyphénylglycine, optiquement pure, à une concentration de 4,05 g/l.

Il va de soi que la présente invention n'a été décrite qu'à titre purement explicatif et nullement limitatif et que toute modification, notamment au niveau des équivalences techniques, pourra y être apportée sans sortir de son cadre.

**Revendications**

**Revendications pour les Etats contractants suivants : AT, BE, CH, DE, FR, GB, GR, IT, LI, LU, NL, SE**

1. Système enzymatique susceptible d'être obtenu par culture d'un micro-organisme tel que l'Agrobacterium identifié par une souche déposée à la Collection Nationale des Micro-organismes à l'Institut Pasteur de Paris sous le N° I-671.

2. Système enzymatique selon la revendication 1, caractérisé par le fait qu'il est obtenu par une culture du micro-organisme défini à la revendication 1 effectuée en présence d'un inducteur d'activité hydantoïnase non métabolisable choisi dans le groupe constitué par l'uracile, le thio-2-uracile, l'aza-6-uracile, la thymine, l'imidazolidinethione, la sec-butylthiohydantoïne, l'orotate de sodium.

3. Application du système enzymatique obtenu selon l'une quelconque des revendications 1 et 2 à la transformation de la D,L-parahydroxyphényl-5 hydantoïne en D-parahydroxyphénylglycine.

4. Application selon la revendication 3, caractérisée par le fait qu'elle est réalisée en présence d'un solvant organique non miscible à l'eau.

**Revendications pour l'Etat contractant suivant : ES**

1. Procédé de préparation d'un système enzymatique caractérisé par le fait que l'on cultive un micro-organisme, tel que l'Agrobacterium identifié par une souche déposée à la Collection Nationale des Microorganismes à l'Institut Pasteur de Paris sous le N° I-167.

2. Procédé de préparation d'un système enzymatique selon la revendication 1, caractérisé par le fait qu'il est obtenu en présence d'un inducteur d'activité hydantoïnase non métabolisable choisi dans le groupe constitué par l'uracile, le thio-2-uracile, l'aza-6-uracile, la thymine, l'imidazolidinethione, la secbutylthiohydantoïne, l'orotate de sodium.

3. Application du système enzymatique obtenu selon l'une quelconque des revendications 1 et 2 à la transformation de la D,L-parahydroxyphényl-5 hydantoïne en D-parahydroxyphénylglycine.

4. Application selon la revendication 3, caractérisée par le fait qu'elle est réalisée en présence d'un solvant non miscible à l'eau.

**Claims**
**Claims for the following Contracting States : AT, BE, CH, DE, FR, GB, GR, IT, LI, LU, NL, SE**

1. An enzyme system obtainable by the culturing of a micro-organism such as the Agrobacterium identified by a strain having the No. I-671 deposited with the National Collection of Micro-organisms at the Pasteur Institute, Paris.

2. An enzyme system according to claim 1, characterized in that it is obtained by a culture of the micro-organism defined in claim 1 effected in the presence of an inducer of nonmetabolizable hydantoinase activity selected from the group formed by uracil, 2-thiouracil, 6-azauracil, thymine, imidazolidinethione, sec.butylthiohydantoin, sodium orotate.

3. Application of the enzyme system according to either of claims 1 and 2 to the conversion of D,L-5-parahydroxyphenyl hydantoin to D-parahydroxyphenyl glycine.

4. Application according to claim 3, characterized in that it is carried out in the presence of an organic solvent immiscible with water.

**Claims for the following Contracting State : ES**

1. A process for the preparation of an enzyme system, characterized in that a micro-organism is cultured such as the Agrobacterium identified by a strain having the No. I-671 deposited with the National Collection of Micro-organisms at the Pasteur Institute, Paris.

2. A process for the preparation of an enzyme system according to claim 1, characterized in that it is obtained by a culture of the micro-organism defined in claim 1 effected in the presence of an inducer of non-metabolizable hydantoinase activity selected from the group formed by uracil, 2-thiouracil, 6-azauracil, thymine, imidazolidinethione, sec.butylthiohydantoin, sodium orotate.

3. Application of the enzyme system according to either of claims 1 and 2 to the conversion of D,L-5-parahydroxyphenyl hydantoin to D-parahydroxyphenyl glycine.

4. Application according to claim 3, characterized in that it is carried out in the presence of an organic solvent immiscible with water.

**Patentansprüche**
**Patentansprüche für folgende Vertragsstaaten : AT, BE, CH, DE, FR, GB, GR, IT, LI, LU, NL, SE**

1. Enzymsystem, das durch Züchtung eines Mikroorganismus, wie Agrobacterium, identifiziert durch einen bei der Collection Nationale des Micro-organismes beim Institut Pasteur, Paris, unter der Nr. I-671 hinterlegten Stamm, erhältlich ist.

2. Enzymsystem nach Anspruch 1, dadurch gekennzeichnet, daß es durch Züchtung des Mikroorganismus, wie in Anspruch 1 definiert, die in Anwesenheit eines nicht-metabolisierbaren Hydantoinase-Aktivitätsinduktors ausgewählt aus der Gruppe bestehend aus Uracil, 2-Thiouracil, 6-Azauracil, Thymin, Imidazolidinthion, sek.Butylthiohydantoin und Natriumorotat durchgeführt wird, erhalten ist.

3. Verwendung des nach einem der Ansprüche 1 und 2 erhaltenen Enzymsystems bei der Überführung von D,L-5-p-Hydroxyphenylhydantoin in D-p-Hydroxyphenylglycin.

4. Verwendung nach Anspruch 3, dadurch gekennzeichnet, daß sie in Anwesenheit eines mit Wasser nicht mischbaren organischen Lösungsmittels durchgeführt wird.

**Patentansprüche für folgenden Vertragsstaat : ES**

1. Verfahren zur Herstellung eines Enzymsystems, dadurch gekennzeichnet, daß ein Mikroorganismus, wie Agrobacterium, identifiziert durch einen bei der Collection Nationale des Micro-organismes beim Institut Pasteur, Paris, unter der Nr. I-671 hinterlegten Stamm, gezüchtet wird.

2. Verfahren zur Herstellung eines Enzymsystems nach Anspruch 1, dadurch gekennzeichnet, daß es in Anwesenheit eines nicht-metabolisierbaren Hydantoinase-Aktivitätsinduktors ausgewählt aus der Gruppe bestehend aus Uracil, 2-Thiouracil, 6-Azauracil, Thymin, Imidazolidinthion, sek.Butylthiohydantoin und Natriumorotat hergestellt wird.

3. Verwendung des nach einem der Ansprüche 1 und 2 erhaltenen Enzymsystems bei der Überführung von D,L-5-p-Hydroxyphenylhydantoin in D-p-Hydroxyphenylglycin.

4. Verwendung nach Anspruch 3, dadurch gekennzeichnet, daß sie in Anwesenheit eines mit Wasser nicht mischbaren organischen Lösungsmittels durchgeführt wird.